# EUROPÄISCHE PATENTSCHRIFT

(11) **EP 1 430 913 B1**
(45) Veröffentlichungstag und Bekanntmachung des Hinweises auf die Patenterteilung: **14.05.2008**
(21) Anmeldenummer: 03023419.9
(22) Anmeldetag: 17.10.2003
(51) Int. Cl.: A61L 24/02, A61L 24/04

(54) **Knochenzement mit verbesserten mechanischen Eigenschaften und Verfahren zu seiner Herstellung**
Bone cement with improved mechanical properties and method for producing it
Ciment osseux avec des propriétés mecaniques améliorées et son procédé de production

(30) Priorität: 20.12.2002 DE 10260918
(43) Veröffentlichungstag der Anmeldung: 23.06.2004
(60) Teilanmeldung: 07090086.5
(73) Patentinhaber: Biomet Deutschland GmbH, 14167 Berlin (DE)
(72) Erfinder: Schilke, Frank, Dipl.-Chem., 64331 Weiterstadt (DE); Nies, Berthold, Dr., 64407 Fränkisch-Crumbach (DE); Jeschke, Brigitte, Dr., 65770 Kelkheim (DE); Koch, Matthias, Dr., 65183 Wiesbaden (DE); Kübelbeck, Armin, Dipl.-Ing., 64625 Bensheim (DE)
(74) Vertreter: Gross, Felix

(56) Entgegenhaltungen:
- EP-A- 0 041 614
- DE-A- 4 029 714
- US-A- 5 795 922

## Beschreibung

Gegenstand der Erfindung ist ein Verfahren zur Herstellung von Knochenzementen aus Polyacrylaten oder Polymethacrylaten unter Verwendung eines Röntgenkontrastmittels, wobei das Röntgenkontrastmittel mit (Meth-) Acrylatgruppen unter vorhergehender Beschichtung mit Melaminharz funktionalisiert wird. Gegenstand der Erfindung ist weiterhin ein Knochenzement gemäß den Ansprüchen 5 bis 8 sowie deren Verwendung zur Verankerung von Prothesenkomponenten im Knochen, zur Versteifung von Knochen, als Dübel von Knochenschrauben oder als Implantat zur Verankerung von Schrauben.

Zur Fixierung von Implantaten im Knochen werden Knochenzemente eingesetzt. Sie bestehen weitgehend aus Polyacrylaten oder Polymethacrylaten, bevorzugt Polymethylmethacrylat (PMMA). Um die Materialien bei Röntgenaufnahmen sichtbar zu machen, werden Röntgenkontrastmittel beigemengt; häufig handelt es sich hierbei um Zirkoniumoxid. Das ZrO₂ geht keine chemische Bindung mit der Poly(meth-) acrylatmatrix ein. Die Teilchen, welche üblicherweise einen Durchmesser von 10 bis 50 µm besitzen, sind lose vom Polymer eingeschlossen. Dies kann zu einer verminderten Bruchfestigkeit des Materials führen.

Aus dem Stand der Technik ist die Zumischung von Röntgenkontraststoffen (i.d.R. ZrO₂ oder BaSO₄) zu einem Pulver, welches aus PMMA- oder PMMA-Copolymer-Perlen und einem Polymerisationsinitiator besteht, bekannt. Die daraus entstehende Pulverkomponente wird zur Polymerisation mit einer Monomerflüssigkeit (i.d.R. Methylmethacrylat) vermischt, in der eine Aktivatorkomponente enthalten ist. Da die genannten Röntgenkontrastmittel eher hydrophil sind, werden sie während der Polymerisation weder physikalisch noch chemisch an die hydrophobe Polymermatrix gebunden.

Diese Teilchen sind daher lose vom Polymer umschlossen, wodurch sich eine Schwächung der Materialfestigkeit ergibt.

Da das Röntgenkontrastmittel nicht an die Polymermatrix gebunden ist, kann es an das umliegende biologische Gewebe abgegeben werden. Auf Grund der Toxizität speziell des Bariumsulfats ist diese Kontamination unerwünscht. So beschreiben Sabokbar und Fujikawa (The Journal of Bone and Joint Surgery, Vol. 79-B, No. 1 (1997)), dass freigesetztes Röntgenkontrastmittel (ZrO₂ und besonders BaSO₄) zu Osteolysen führen kann. Bei PMMA-Zement ohne Röntgenkontrastmittel wurde dieser Effekt nicht beobachtet.

Die Freisetzung der harten Zirkonium-Partikel an der Grenze zwischen Zement und Metallimplantat gilt darüber hinaus als eine Ursache der Prothesenlockerung durch mechanischen Abrieb.

Die DE 4029714 (Draenert) beschreibt ein Verfahren bei dem Röntgenkontrastmittel (Füllerpartikel) in eine Schmelze des PMMA-Polymers eingearbeitet werden und anschließend durch Fällung dieser Schmelze in einem Fällbad Polymerperlen erzeugt werden, die das Röntgenkontrastmittel zumindest teilweise einbetten. Gefällte Polymerperlen haben auf Grund ihrer anderen Oberflächenstruktur jedoch auch andere mechanische und Quellungs-Eigenschaften als die im Knochenzement üblicherweise verwendeten Perlpolymerisate. Nachteilig ist, dass sich bei diesem Verfahren ein Teil des Röntgenkontrastmittels an der Oberfläche der PMMA-Perlen befindet, so dass der mech. Abrieb dadurch nicht behoben wird.

Die EP 089782 (U.S. Surgical Corp.) beschreibt ein Verfahren zur Herstellung einer porösen implantierbaren Prothese, bei dem ein Röntgenkontrastmittel (Bariumsulfat) in Polymerteilchen eingearbeitet wird. Im Gegensatz zum erfindungsgemäßen Verfahren wird das Röntgenkontrastmittel nicht in PMMA einpolymerisiert, sondern auf bestehende PMMA-Teilchen mittels 4-7 % Hydroxyethylmethacrylat aufgeklebt. Diese BaSO₄ / Poly(hydroxyethyl- methacrylat)- Beschichtung erfüllt nicht die Anforderungen, die an einen Knochenzement gestellt werden. Es wird daher lediglich als pulverförmiges röntgendichtes Füllmaterial für Knochendefekte eingesetzt.

Die U.S. 5,795,922 (Demian et al.) offenbart ein Verfahren zur Herstellung von Polymerperlen mit eingebetteten Röntgenkontrastmittel. Die hier beschriebene Fällung von PMMA auf Bariumsulfatpartikel aus einem Lösungsmittel und anschließende Einpolymerisation in PMMA-Perlen unterscheidet sich grundlegend von dem erfindungsgemäßen Verfahren.

Die EP 581 387 (BMS) beschreibt ein Verfahren, bei dem durch Fällung oder Aufschmelzen eines Polymers auf Röntgenkontrastmittelpartikel hergestellte Polymerpartikel in einem weiteren Schritt in Polymerperlen eingebettet werden.

Die EP 041614 (Bayer AG) offenbart ein Verfahren, bei dem das Röntgenkontrastmittel Bariumsulfat mittels Ausfällung von einem in einem Lösungsmittel gelösten (Meth-) Acrylsäure-Copolymer beschichtet wird. Dieses Material ist jedoch aufgrund seiner chemischen Zusammensetzung für die Verwendung von Knochenzementen ungeeignet.

Die U.S. 4,500,658 (Austenal Int. , Inc.) beschreibt ein Verfahren zur Herstellung von radioopaken Acryl-Partikeln in Perlenform, wobei die Kompatibilisierung des Röntgenkontrastmittels während der Perlpolymerisation in situ mittels geeigneter Silanierungsmittel durchgeführt wird.

Aufgabe der vorliegenden Erfindung war es ein Verfahren sowie einen Knochenzement zur Verfügung zu stellen, der die vorher genannten Nachteile aus dem Stand der Technik vermeidet.

Diese Aufgabe wird durch das Verfahren und den Knochenzement gemäß der Erfindung gelöst.
Die erfindungsgemäße Funktionalisierung anorganischer Füllmaterialien wie ZrO₂ und BaSO₄ mit (Meth-) Acrylatgruppen erlaubt eine chemische Verbindung mit dem umgebenden Polymer des ausgehärteten Knochenzements. Die Beimengung ist damit nicht länger lose in der Matrix eingebaut, sondern vielmehr ein fester Bestandteil derselben. Dies führt dazu, dass Mikrorisse nicht länger entlang der Grenze zwischen den anorganischen Teilchen und der Matrix propagieren können. Weiterhin wird durch diese Modifikation die Abriebfestigkeit des Zements erhöht, die Freisetzung der Füllmaterialien ins umgebene Gewebe vermindert und die dauerhafte Kontamination der PE-Laufflächen von Implantaten mit Röntgenkontrastmitteln verhindert. Es hat sich nun überraschenderweise herausgestellt, dass auch die Einmischbarkeit des Röntgenkontrastmittels in den Zement durch die Modifikation verbessert wird. Um Acrylatgruppen auf das Röntgenkontrastmittel aufbringen zu können, ist eine vorhergehende Beschichtung mit Melaminharz als reaktive Zwischenschicht notwendig. Melaminharzbeschichtete Partikel weisen überraschenderweise eine deutlich höhere Reaktivität auf als unbeschichtetes Material.

Das erfindungsgemäße Verfahren zur Herstellung von Knochenzementen aus Polyacrylaten oder Polymethacrylaten unter Verwendung eines Röntgenkontrastmittels ist gekennzeichnet durch die Funktionalisierung dieses Kontrastmittels mit (Meth-) Acrylatgruppen gemäß folgender Schritte:
a) Suspendieren des festen Röntgenkontrastmittels in Wasser und anschließendem Erhitzen.
b) Zugabe von Melamin-Formaldehyd-Harz sowie einer Säure, vorzugsweise Ameisensäure
c) Waschen und Trocknen der melaminbeschichteten Teilchen.
d) Zugabe von (Meth)-Acrylsäurechlorid unter Rühren
e) Waschen und Trocknen des mit reaktiven (Meth)-Acrylatgruppen funktionalisierten Röntgenkontrastmittels.

Als Knochenzement werden Polyacrylate oder Polymethacrylate, insbesondere PMMA eingesetzt.

Beim Röntgenkontrastmittel, welches die Aufgabe hat, Röntgenstrahlen zu absorbieren und damit das Material bei entsprechenden Aufnahmen sichtbar zu machen, spielt nur die Menge (Masse) des eingesetzten Materials eine Rolle. Sie beträgt vorzugsweise zwischen 8 und 16 Gew% der Masse des Zementpulvers.
Als Röntgenkontrastmittel können herkömmliche Partikel im Bereich von 05. - 50 µm Durchmesser verwendet werden. Bevorzugt ist jedoch nanoskaliges Material mit einer Partikelgröße von 5 bis 500 nm, welches zur weiteren Verbesserung der Materialeigenschaften gewählt wird. Dadurch kommt es zu einer deutlich gleichmäßigeren Verteilung der anorganischen Partikel in der Polymermatrix. Bei gleicher Beigabemenge sind die Fehlstellen um den Faktor 500-1000 kleiner, wodurch ihr negativer Einfluss auf die Festigkeit des Materials deutlich geringer wird.

Es werden vorzugsweise ZrO₂, BaSO₄, bismuthaltige oder jodhaltige Kontrastmittel verwendet. Besonders bevorzugt ist ZrO₂.

Die erfindungsgemäßen Knochenzemente lassen sich zur Verankerung von Prothesenkomponenten im Knochen und zur Versteifung von Knochen verwenden. Ferner kann er auch als Dübel von Knochenschrauben bzw. als Implantat zur Verankerung von Schrauben verwendet werden.

### Beispiel:

Funktionalisierung von Zirkoniumoxid mit Acrylatgruppen:
250 g Zirkoniumoxid (MERCK 100757) werden in 4 I Wasser suspendiert und auf 70 °C erwärmt. Dazu kommen 80g Madurit^{®} SMW818 (=Melamin-Formaldehydharz) sowie 150 ml Ameisensäure (w= 2%). Die Mischung wird weitere 30 min bei 70 °C gerührt, bevor man sie auf Raumtemperatur abkühlen lässt. Die melaminbeschichteten Teilchen werden gewaschen und getrocknet.
200g dieses Pulvers werden in 150 ml Acrylsäurechlorid über Nacht bei Raumtemperatur gerührt, von der verbleibenden Acrylsäure abgetrennt, mit Natronlauge und Wasser neutral gewaschen und im Vakuum bei Raumtemperatur getrocknet.
Man erhält ein frei fließendes Pulver von Zirkoniumoxid mit reaktiven Acrylatgruppen auf der Oberfläche, die sich radikalisch mit Acrylaten copolymerisieren lassen.

## Patentansprüche

1. Verfahren zur Herstellung von Knochenzementen aus Polyacrylaten oder Polymethacrylaten unter Verwendung eines Röntgenkontrastmittels,
**dadurch gekennzeichnet, dass**
a) das Röntgenkontrastmittel mit (Meth-) Acrylatgruppen funktionalisiert wird, wobei
b) der Funktionalisierung des Röntgenkontrastmittels mit (Meth-) Acrylatgruppen eine Beschichtung mit Melamin-Formaldehydharz vorhergeht.

2. Verfahren nach Anspruch 1, **dadurch gekennzeichnet, dass** die Funktionalisierung des Röntgenkontrastmittels mit (Meth-) Acrylatgruppen folgende Schritte umfasst:
a) Suspendieren des festen Röntgenkontrastmittels in Wasser und anschliessendem Erhitzen.
b) Zugabe von Melamin-Formaldehydharz sowie einer Säure
c) Waschen und Trocknen der Melamin-Formaldehydharzbeschichteten Teilchen.
d) Zugabe von (Meth-) Acrylsäurechlorid unter Rühren
e) Waschen und Trocknen des mit reaktiven (Meth-) Acrylatgruppen funktionalisierten Röntgenkontrastmittels.

3. Verfahren nach einem der Ansprüche 1 und/oder 2, **dadurch gekennzeichnet, dass** als Röntgenkontrastmittel ein nanoskaliges Kontrastmittel mit einer Partikelgrösse von 5 bis 500 nm verwendet wird.

4. Verfahren nach einem der Ansprüche 1 bis 3, **dadurch gekennzeichnet, dass** vom Röntgenkontrastmittel eine Menge zwischen 8 und 16 Gew. % der Masse des Zementpulvers eingesetzt werden.

5. Knochenzement bestehend aus Polyacrylaten oder Polymethacrylaten und einem Röntgenkontrastmittel,
**dadurch gekennzeichnet,**
a) **dass** das nanoskalige Röntgenkontrastmittel mit einer Partikelgrösse von 5 bis 500 nm mit (Meth-) Acrylatgruppen funktionalisiert ist, wobei
b) der Funktionalisierung des Röntgenkontrastmittels mit (Meth-) Acrylatgruppen eine Beschichtung mit Melamin-Formaldehydharz vorhergeht.

6. Knochenzement nach Anspruch 5, **dadurch gekennzeichnet, dass** die Menge an Röntgenkontrastmittel zwischen 8 und 16 Gew.% der Masse des Zementpulvers beträgt.

7. Knochenzement nach einem der Ansprüche 5 und/oder 6, **dadurch gekennzeichnet, dass** das Röntgenkontrastmittel Zirkoniumdioxid (ZrO₂), Bariumsulfat (BaSO₄), bismuthaltig oder jodhaltig ist.

8. Verwendung des Knochenzements nach einem der Ansprüche 5 bis 7 zur Herstellung eines Arzneimittels zur Verankerung von Prothesenkomponenten im Knochen, zur Versteifung von Knochen, als Dübel von Knochenschrauben oder als Implantat zur Verankerung von Schrauben.

## Claims

1. Process for the preparation of bone cements from polyacrylates or polymethacrylates by using an X-ray contrast medium,
**characterized in that**
a) the X-ray contrast medium is functionalized with (meth)acrylate groups, wherein
b) the functionalizing of the X-ray contrast medium with the (meth)acrylate groups is preceded by coating with melamine-formaldehyde resin.

2. Process according to Claim 1, **characterized in that** the functionalizing of the X-ray contrast medium with meth(acrylate) groups comprises the steps of:
a) suspending the solid X-ray contrast medium in water and subsequent heating,
b) adding a melamine-formaldehyde resin and also an acid
c) washing and drying the melamine-formaldehyde resin coated particles
d) adding (meth)acryloyl chloride with stirring
e) washing and drying the X-ray contrast medium functionalized with reactive (meth)acrylate groups.

3. Process according to either of Claims 1 and/or 2, **characterized in that** the X-ray contrast medium used is a nanoscale contrast medium having a particle size of 5 to 500 nm.

4. Process according to any one of Claims 1 to 3, **characterized in that** the X-ray contrast medium is used in an amount between 8 and 16% be weight of the mass of the cement powder.

5. Bone cement consisting of polyacrylates or polymethacrylates and an X-ray contrast medium,
**characterized in that**
a) the nanoscale X-ray contrast medium having a particle size of 5 to 500 nm is functionalized with (meth)acrylate groups, wherein
b) the functionalizing of the X-ray contrast medium with the (meth)acrylate groups is preceded by coating with melamine-formaldehyde resin.

6. Bone cement according to Claim 5, **characterized in that** the amount of X-ray contrast medium is between 8 and 16% by weight of the mass of the cement powder.

7. Bone cement according to either of Claims 5 and/or 6, **characterized in that** the X-ray contrast medium is zirconium dioxide (ZrO₂), barium sulphate (BaSO₄), bismuth containing or iodine containing.

8. Use of the bone cement according to any one of Claims 5 to 7 in the manufacture of a medicament for anchoring prosthesis components in bone, for stiffening bone, as bone screw fixing plug or as implant for anchoring screws.

## Revendications

1. Procédé pour la préparation de ciments osseux à partir de polyacrylates ou de polyméthacrylates avec utilisation d'un agent de contraste aux rayons X, **caractérisé en ce que**
a) l'agent de contraste aux rayons X est fonctionnalisé avec des groupes (méth)acrylate, où
b) la fonctionnalisation de l'agent de contraste aux rayons X avec des groupes (méth)acrylate est précédée d'un revêtement avec une résine de mélamine-formaldéhyde.

2. Procédé selon la revendication 1, **caractérisé en ce que** la fonctionnalisation de l'agent de contraste aux rayons X avec des groupes (méth)acrylate comprend les étapes suivantes :
a) mise en suspension de l'agent de contraste aux rayons X solide dans l'eau et chauffage consécutif
b) addition de résine de mélamine-formaldéhyde ainsi que d'un acide
c) lavage et séchage des particules revêtues de résine de mélamine-formaldéhyde
d) addition de chlorure de l'acide (méth)acrylique sous agitation
e) lavage et séchage de l'agent de contraste aux rayons X fonctionnalisé avec les groupes (méth)acrylate réactifs.

3. Procédé selon l'une quelconque des revendications 1 et/ou 2, **caractérisé en ce qu'**on utilise comme agent de contraste aux rayons X un agent de contraste d'une taille à l'échelle du nanomètre présentant une grosseur de particule de 5 à 500 nm.

4. Procédé selon l'une quelconque des revendications 1 à 3, **caractérisé en ce qu'**on utilise l'agent de contraste aux rayons X en une quantité entre 8 et 16% en poids de la masse de poudre de ciment.

5. Ciment osseux constitué par des polyacrylates ou des polyméthacrylates et un agent de contraste aux rayons X,
**caractérisé en ce que**
a) l'agent de contraste aux rayons X d'une taille à l'échelle du nanomètre présentant une grosseur de particule de 5 à 500 nm est fonctionnalisé avec des groupes (méth)acrylate, où
b) la fonctionnalisation de l'agent de contraste aux rayons X avec des groupes (méth)acrylate est précédée d'un revêtement avec une résine de mélamine-formaldéhyde.

6. Ciment osseux selon la revendication 5, **caractérisé en ce que** la quantité d'agent de contraste aux rayons X est comprise entre 8 et 16% en poids de la masse de poudre de ciment.

7. Ciment osseux selon l'une quelconque des revendications 5 et/ou 6, **caractérisé en ce que** l'agent de contraste aux rayons x est le dioxyde de zirconium (ZrO₂), le sulfate de baryum (BaSO₄), contient du bismuth ou de l'iode.

8. Utilisation du ciment osseux selon l'une quelconque des revendications 5 à 7 pour la préparation d'un médicament destiné à l'ancrage de composants de prothèse dans des os, pour rigidifier des os, comme cheville pour vis dans les os ou comme implant pour l'ancrage de vis.
